# EUROPÄISCHE PATENTSCHRIFT

(11) **EP 3 441 936 B1**
(45) Veröffentlichungstag und Bekanntmachung des Hinweises auf die Patenterteilung: **21.04.2021**
(21) Anmeldenummer: 17185866.5
(22) Anmeldetag: 11.08.2017
(51) Int. Cl.: G06T 7/00

(54) **VERFAHREN ZUR AUSWERTUNG VON BILDDATEN EINES PATIENTEN NACH EINEM MINIMALINVASIVEN EINGRIFF, AUSWERTEEINRICHTUNG, COMPUTERPROGRAMM UND ELEKTRONISCH LESBARER DATENTRÄGER**
METHOD FOR EVALUATING IMAGE DATA OF A PATIENT AFTER A MINIMALLY INVASIVE PROCEDURE, EVALUATION DEVICE, COMPUTER PROGRAM AND ELECTRONICALLY READABLE DATA CARRIER
PROCÉDÉ D'ÉVALUATION DE DONNÉES D'IMAGE D'UN PATIENT SOUMIS À UNE INTERVENTION CHIRURGICALE PEU INVASIVE, DISPOSITIF D'ÉVALUATION, PROGRAMME INFORMATIQUE ET SUPPORT DE DONNÉES LISIBLE PAR VOIE ÉLECTRONIQUE

(43) Veröffentlichungstag der Anmeldung: 13.02.2019
(73) Patentinhaber: Siemens Healthcare GmbH, 91052 Erlangen (DE)
(72) Erfinder: Sühling, Michael, 91052 Erlangen (DE); Flohr, Thomas, 91486 Uehlfeld (DE); Reichelt, Stefan, 96049 Bamberg (DE)

(56) Entgegenhaltungen:
- DE-A1-102014 212 089
- US-A1- 2007 201 735
- US-A1- 2015 087 957
- MADAY PETER ET AL: "Imaging as a Surrogate for the Early Prediction and Assessment of Treatment Response through the Analysis of 4-D Texture Ensembles (ISEPARATE)", NETWORK AND PARALLEL COMPUTING; [LECTURE NOTES IN COMPUTER SCIENCE; LECT.NOTES COMPUTER], SPRINGER INTERNATIONAL PUBLISHING, CHAM, Bd. 6533 Chap.16, Nr. 558, 20. September 2010 (2010-09-20), Seiten 164-173, XP047442062, ISSN: 0302-9743 ISBN: 978-3-642-01969-2

## Beschreibung

Die Erfindung betrifft ein Verfahren zur Auswertung von Bilddaten eines Patienten nach einem minimalinvasiven Eingriff an einer Läsion, insbesondere einer Tumorablation an einem Tumor, wobei ein präinterventioneller, die Läsion zeigender Bilddatensatz und ein intra- und/oder postinterventioneller zweiter, das Eingriffsgebiet zeigender Bilddatensatz ausgewertet werden. Daneben betrifft die Erfindung eine Auswerteeinrichtung, ein Computerprogramm und einen elektronisch lesbaren Datenträger.

In der Vergangenheit wurden bereits eine Vielzahl minimalinvasiver Interventionstechniken zur Behandlung von Läsionen, insbesondere Tumoren, innerhalb eines Patienten vorgeschlagen. Hierbei wird üblicherweise ein minimalinvasives, medizinisches Instrument, beispielsweise eine Behandlungsnadel und/oder ein Katheter, zur Läsion geführt, woraufhin dort entsprechend der behandelnde Eingriff, also die Intervention, erfolgt. Ein Beispiel hierfür ist die ablative Behandlung von Tumoren (Tumorablation), wobei sowohl primäre Tumoren als auch Metastasen in im Wesentlichen allen Bereichen des Körpers behandelt werden können. Die Tumorablation ist eine Technik, in der kontrolliert Zellen und Gewebe zerstört werden sollen, insbesondere durch Temperaturbehandlung (Erhitzen oder Abkühlen). Häufig eingesetzte Behandlungstechniken umfassen die perkutane Hochfrequenzablation (Radio Frequency Ablation - RFA), die Mikrowellenablation (Microwave Ablation - MWA) und die Kryoablation.

Minimalinvasive Eingriffe, insbesondere Tumorablationen, werden dabei häufig unter Bildüberwachung durchgeführt. Dabei werden Bilddaten des Eingriffsgebiets und der zu behandelnden Läsion mittels einer Bildaufnahmeeinrichtung, beispielsweise einer Röntgeneinrichtung, aufgenommen. Insbesondere wurde auch die Computertomographie (CT) als Hilfsmittel zur Navigation bei minimalinvasiven Eingriffen vorgeschlagen.

Neben den während und/oder zum Abschluss des minimalinvasiven Eingriffes entstehenden Bilddatensätzen (intra- und/oder postinterventionelle Bilddaten) liegen dem minimalinvasiven Eingriff häufig auch präinterventionelle Bilddaten zugrunde, beispielsweise dreidimensionale Planungsdatensätze, die die Läsion hinreichend deutlich zeigen und bei der Bildüberwachung der minimalinvasiven Eingriffs auch als Überlagerungsbilder eingesetzt werden können. Entsprechende Techniken zur Unterstützung einer einen Eingriff durchführenden Person durch Bildüberwachung sind im Stand der Technik bereits vielfältig beschrieben worden.

Problematisch bei solchen minimalinvasiven, eine Läsion behandelnden Eingriffen ist, dass der Erfolg bzw. Misserfolg des minimalinvasiven Eingriffes aufgrund der aufgenommenen Bilddaten und/oder sonstigen Informationen heute nicht verlässlich beurteilt werden kann. In der klinischen Praxis wird das Ergebnis der Behandlung üblicherweise visuell und qualitativ durch eine den Eingriff durchführende Person beurteilt, wobei als Basis Bilddaten dienen, die während oder unmittelbar nach dem minimalinvasiven Eingriff aufgenommen wurden, mithin auch intra- und/oder postinterventionellen Bilddaten. Nachdem die Beurteilung durch Betrachtung der Bilddaten seitens einer Person vorgenommen wird, ist das Beurteilungsergebnis als subjektiv und personenabhängig zu bezeichnen. In den Bilddatensätzen, beispielsweise CT-Scans, werden wechselnde Bildeindrücke herangezogen, beispielsweise Begrenzungen nekrotischen Gewebes, Kavitationen oder sogenannte "Ground Glass Opacities" (GGO) um die Läsion. Ein Tumordurchmesser kann abnehmen oder sich nicht während des Eingriffs verändern, abhängig vom Tumortyp. Das Ergebnis sind Situationen, in denen die Behandlung "übertrieben" wird, mithin eine zu starke Behandlung vorgenommen wird, die ein unnötiges Risiko bzw. eine unnötige Komplikation für den Patienten darstellt. Auf der anderen Seite können dann, wenn die Behandlung nicht hinreichend ist, verbleibende Anteile des Tumors zu einem Fortschreiten der Krankheit führen, was ebenso ein Risiko für den Patienten darstellt.

Mithin ermöglich der heutige Stand der Technik eine verlässliche Beurteilung des Behandlungserfolgs erst deutlich nach dem Durchführen des minimalinvasiven Eingriffs, beispielsweise basierend auf Folge-Bildaufnahmen. Problematisch hierbei ist, dass kurzfristig angesetzte, effektive Korrekturmaßnahmen, insbesondere weitere ablative Eingriffe, auf dieser Zeitskala nicht mehr möglich sind.

Der Erfindung liegt daher die Aufgabe zugrunde, eine verbesserte Beurteilung des Behandlungserfolgs eines minimalinvasiven Eingriffs zeitlich näher an dem Eingriff, insbesondere während und/oder unmittelbar nach dem Eingriff, zu schaffen.

Zur Lösung dieser Aufgabe ist bei einem Verfahren der eingangs genannten Art erfindungsgemäß vorgesehen, dass aus den Bilddatensätzen wenigstens teilweise automatisch Eigenschaften der Bilddaten beschreibende Eigenschaftsdaten abgeleitet werden, welche einem mit Trainingsdaten, denen eine Grundwahrheit zugeordnet ist, anderer Patienten trainierten Auswertungsalgorithmus der künstlichen Intelligenz als Eingangsdaten bereitgestellt werden, welcher aus den Eigenschaftsdaten eine die Veränderung der Läsion und/oder im Eingriffsgebiet durch den Eingriff beschreibende Veränderungsinformation ermittelt.

Erfindungsgemäß wird also ein datengetriebenes Vorgehen vorgeschlagen, um eine objektive Beurteilung des Behandlungsergebnisses früh während bzw. nach dem Eingriff zu ermöglichen, um frühes und optimales Patientenmanagement zu erlauben. Mit der Veränderungsinformation wird eine objektive, die stattgefundene Veränderung im Eingriffsgebiet bzw. konkret an der Läsion beschreibende Auswertungsinformation bereitgestellt, die eine hervorragende Grundlage für eine Beurteilung und mithin eine daraus folgende diagnostische Aussage, die von einer behandelnden Person getroffen werden muss, bildet. Dabei hat sich gezeigt, dass Methoden der künstlichen Intelligenz bzw. des maschinellen Lernens sich besonders vorteilhaft in diesem Gebiet einsetzen lassen. Diese Vorgehensweisen kommen ohne explizite physikalische Modellierung aus, beispielsweise ohne physikalische Modelle, die die Wärmeübertragung basierend auf Differentialgleichungen beschreiben. Durch rein datengetriebenes Vorgehen, bei dem sich insbesondere der im Training des Auswertungsalgorithmus verwendete Trainingsalgorithmus die physikalischen Zusammenhänge und Korrelationen selbst erschließt, kann auf subjektive und/oder gegebenenfalls falsche Annahmen im Wesentlichen verzichtet werden.

Dabei können verschiedene geeignete konkrete Auswertungsalgorithmen der künstlichen Intelligenz eingesetzt werden. Vorzugsweise kann als Auswertungsalgorithmus ein neuronales Netz und/oder ein Random-Forest-Algorithmus verwendet werden.

Eine besonders vorteilhafte Ausgestaltung der vorliegenden Erfindung sieht vor, dass wenigstens ein Teil der als Eingangsdaten zu verwendenden Eigenschaftsdaten und/oder Eigenschaften des Auswertungsalgorithmus durch einen den Auswertungsalgorithmus trainierenden Trainingsalgorithmus, insbesondere einen Deep-Learning-Algorithmus, definiert werden. Werden Techniken des sogenannten "Deep Learning" verwendet, werden dem Trainingsalgorithmus mithin unmittelbar die Bilddaten bereitgestellt, so dass dieser die darin vorhandenen physikalischen Zusammenhänge lernen kann und die diese Zusammenhänge bzw. Korrelationen am besten beschreibenden Eigenschaften der Bilddaten genauso auswählen kann wie der Auswertungsalgorithmus geeignet abgestimmt auf diese konfiguriert werden kann. Insbesondere ist es denkbar, dass der Trainingsalgorithmus die geeigneten, aus den Bilddaten abzuleitenden Eigenschaften zumindest teilweise selbst definiert. Derartige Eigenschaften können auch als "Deep-Learning-Eigenschaften" bezeichnet werden.

Alternativ oder zusätzlich ist es im Rahmen der vorliegenden Erfindung auch denkbar, dass wenigstens ein Teil der als Eingangsdaten zu verwendenden Eigenschaftsdaten aus einer vorgegebenen Menge an Kandidateneigenschaften beschreibenden Kandidatendaten ausgewählt werden. Sind beispielsweise aus Untersuchungen, wissenschaftlichen Analysen und dergleichen bereits Kandidateneigenschaften bekannt, von welchen man sich einen starken Zusammenhang mit der Veränderungsinformation erhofft, können einem Trainingsalgorithmus, insbesondere auch dem Deep-Learning-Trainingsalgorithmus, diese entsprechend angeboten werden, was gegebenenfalls den Trainingsprozess insgesamt vereinfachen kann, wenn hier bereits geeignete Eingangsdaten innerhalb der Kandidatendaten aufgefunden werden können.

Eine besonders vorteilhafte Weiterbildung der vorliegenden Erfindung sieht vor, dass als Eingangsdaten des Auswertungsalgorithmus und/oder zur Ermittlung der Eigenschaftsdaten zusätzlich den Bilddatensätzen zugeordnete, zur Aufnahme von Bilddaten der Bilddatensätze verwendete Aufnahmeparameter verwendet werden. Bei der Bildinterpretation ist es häufig nicht irrelevant, wie das entsprechende Bild entstanden ist, beispielsweise mit welcher Intensität Konturen in einem Röntgenbild abgegeben werden, welche Kontraste durch die Bilddaten beispielsweise bei einem Magnetresonanzbild wiedergegeben werden und dergleichen. Dies kann sowohl Einfluss auf die konkrete Ermittlung der Eigenschaftsdaten für diese Bilddaten haben als auch für die Arbeit des Auswertungsalgorithmus selbst, da dann die insgesamte Bandbreite von möglichen Aufnahmeparametern für die Bilddaten abgedeckt und in den durch den Auswertungsalgorithmus beschriebenen Zusammenhängen berücksichtigt werden können. Allgemeine Aufnahmeparameter betreffen beispielsweise die Aufnahmezeit, Kontrastmitteleigenschaften, den abgebildeten Bereich des Patienten, die verwendete Bildaufnahmemodalität bzw. Bildaufnahmeeinrichtung, Aufnahmegeometrieparameter und dergleichen. Speziell für Röntgenbilddaten sind hierbei insbesondere auch die Dosis und/oder das Energiespektrum der Röntgenstrahlen zu nennen. Besonders vorteilhaft ist es ferner, wenn wenigstens ein Teil der Bilddaten Multienergie-Röntgenbilddaten mit zugeordneten Energiespektreninformationen umfasst. Die Mehrenergie-Röntgenbildgebung, insbesondere die Dualenergie-Röntgenbildgebung, liefert aufgrund der teilweise von der Strahlenenergieverteilung abhängigen Absorptionseigenschaften unterschiedlicher Materialien eine große Menge zusätzlicher Informationen, die besonders vorteilhaft im Rahmen sowohl des maschinellen Lernens als auch der Anwendung des Ausführungsalgorithmus in ihrer Gänze ausgenutzt werden können. Dabei ist insbesondere die sogenannte Spektral-CT zu nennen. Durch die Einbettung in ein künstliche Intelligenz verwendendes datengetriebenes System kann diese Vielzahl von Informationen möglichst weitgehend berücksichtigt und genutzt werden.

Im Rahmen der vorliegenden Erfindung können eine Vielzahl von Eigenschaftsdaten verwendet und in Betracht gezogen werden. Eine Gruppe von Eigenschaftsdaten, die sich als geeignet erwiesen hat, umfasst Identifikations- und Positionsinformationen zu der Läsion und/oder die Läsion umgebenden anatomischen Strukturen, insbesondere als annotierte Regionen, und/oder ein verwendetes, in den Bilddaten sichtbares Instrument betreffende Instrumentinformationen. Derartige Eigenschaftsdaten können, wie im Stand der Technik vielfältig beschrieben, durch Verwendung moderner Algorithmen zumindest teilweise oder auch vollständig automatisch aus den Bilddaten abgeleitet werden. Identifikations- und Positionsinformationen zu anatomischen Strukturen geben beispielsweise die Art und Ausdehnung dieser anatomischen Strukturen, die die Zielläsion umgeben, an, bevorzugt für alle durch Bilddaten abgedeckte Zeitpunkte. Beispielsweise können hierbei die Position und Ausdehnung von Organen, wie Leber oder Lunge, beschrieben werden, wobei eine besonders vorteilhafte Ausgestaltung bei der thermischen Tumorablation und/oder sonstigen thermischen Behandlungen vorsieht, dass als spezielle Eigenschaftsdaten bei thermischen Behandlungen an der Läsion Wärmeleitungswege beschreibende Wärmeleitungsinformationen berücksichtigt werden. Insbesondere können hierdurch Blutgefäße und/oder Luftwege beschrieben werden, die als Wärmesenken während des minimalinvasiven Eingriffs wirken können. Optional können Eigenschaftsdaten in diesem Kontext auch biophysikalische Gewebeeigenschaften, die den identifizierten anatomischen Strukturen zugeordnet sind, umfassen, die ebenso als Teil des gesamten Eigenschaftssatzes berücksichtigt werden können. Ähnliche Ausführungen betreffen auch die Läsion, wobei es hier besonders vorteilhaft ist, wenn alle durch Bilddaten abgedeckten Zeitpunkte berücksichtigt werden, mithin für alle diese Zeitpunkte die Identifikation und die Ausdehnung der Läsion als Zielregion bekannt sind. Auch hierfür wurden bereits Algorithmen vorgeschlagen, die diese Information automatisch bestimmen können.

Die Ermittlung der Eigenschaftsdaten kann auch eine Instrumentendetektion, falls das Instrument in den Bilddaten zu sehen ist, und -lokalisierung umfassen, was in Geometriedaten und der Position von Instrumenten und/oder sonstigen Einrichtungen innerhalb der Bilddaten resultiert. Geometriedaten zu dem Instrument können dabei zweckmäßigerweise aus den Eingriff beschreibenden Eingriffsdaten (Intervention Procedure Data) abgeleitet werden, beispielsweise aus einem CAD-Modell des Instruments.

Eine weitere Gruppe möglicher Eigenschaftsdaten umfasst Form- und Textureigenschaften der Bilddaten beschreibende Eigenschaftsdaten, die im Rahmen der vorliegenden Erfindung ebenso verwendet werden können. Dabei kann vorgesehen sein, dass durch die Eigenschaftsdaten beschriebene Eigenschaften wenigstens ein Element der folgenden Gruppe umfassen: Aus wenigstens einem Intensitätshistogramm abgeleitete Kenngröße, insbesondere Mittelwerte, Entropie und Zentralmomente; Formeigenschaften, beispielsweise Größe, Dichte, Exzentrizität, Sphärizität und/oder Kompaktheit; Bildtextureigenschaften wie Intra-Tumor-Heterogenität, Gabor-Jets und Ergebnisse von LoG, Kantenfiltern und/oder Wavelets; Bildvoxelbeziehungen, beispielsweise die maximale Länge gleicher Bildwerte, Angaben zu Graustufen in der Umgebung eines Bildelements und/oder Abhängigkeitsmatrizen; Fraktaleigenschaften; und Eigenschaften der Spektral-CT, beispielsweise eine Jod-Karte und/oder virtuelle Nativbilder.

In einer besonders vorteilhaften Weiterbildung der vorliegenden Erfindung kann vorgesehen sein, dass als Eingangsdaten des Auswertungsalgorithmus und/oder zur Ermittlung der Eigenschaftsdaten zusätzlich den Patienten betreffende Patienteninformationen und/oder Diagnoseinformationen und/oder den Eingriff beschreibende Eingriffsinformationen verwendet werden. Die Auswertung kann mithin durch weitere Berücksichtigung von Nicht-Bild-Informationen nochmals deutlich verbessert werden. Derartige Nicht-Bild-Informationen können als Eingriffsinformationen insbesondere die Eingriffsart (beispielsweise RF-Ablation, Mikrowellenablation oder Kryoablation), die Eingriffszeitpunkte (beispielsweise Anfangs- und Endpunkte der Anwendung eines Instruments), die eingebrachte Energie, gemessene Temperaturen (beispielsweise durch zusätzlich eingebrachte Temperaturmesseinrichtungen), Instrumentengeometrien und -eigenschaften und dergleichen und/oder als Patientendaten beispielsweise Informationen wie Blutgerinnungseigenschaften des Patienten, gegebene Medikation, Geschlecht des Patienten, Alter des Patienten und/oder Größe des Patienten umfassen.

Zweckmäßigerweise können den Eigenschaftsdaten und/oder weiteren Eingangsdaten jeweils eine zeitliche und/oder räumliche Einordnung erlaubende Einordnungsinformationen zugeordnet werden. Spätestens beim Zusammenstellen der Eingangsdaten, beispielsweise als ein Eingangsdatenvektor, für den Auswertungsalgorithmus ist es zweckmäßig, den einzelnen Eigenschaften und/oder ggf. den weiteren Informationen, womöglich, wenigstens teilweise zeitliche und/oder räumliche Einordnungsinformationen zuzuordnen, insbesondere also zuzufügen, so dass auch diese von dem Auswertungsalgorithmus berücksichtigt werden können. Beispielsweise können einzelnen Eigenschaftsdaten zu der Läsion die Zeitpunkte zugordnet werden, zu denen die Bilddaten, aus denen diese Eigenschaftsdaten abgeleitet wurden, aufgenommen wurden und dergleichen. Es kann mithin mit anderen Worten ein räumlich-zeitliches Kartieren der Eigenschaften und ggf. der weiteren Informationen über die Zeitpunkte, zu denen Bilddaten und gegebenenfalls weitere Informationen vorliegen, erfolgen.

Allgemein kann im Rahmen der vorliegenden Erfindung vorgesehen sein, dass die Veränderungsinformation und/oder daraus abgeleitete Ausgabeinformationen an einen Benutzer ausgegeben werden. Eine zweckmäßige Ausgestaltung in diesem Kontext sieht vor, dass aus der Veränderungsinformation eine wenigstens einem der Bilddaten überlagert darzustellende Auswertungsdarstellung erzeugt und ausgewertet wird. Auf diese Weise können wichtige Veränderungen zusätzlich zu den Bilddaten angezeigt werden, um diese besser verständlich zu gestalten. Eine besonders vorteilhafte Weiterbildung der Erfindung kann jedoch auch vorsehen, dass als Auswertungsdarstellung bzw. weitere Auswertungsdarstellung eine visuelle Wiedergabe von wenigstens einem Teil der Bilddaten überlagert darzustellenden Eigenschaftsdaten zu erzeugen und entsprechend überlagert auszugeben. Auf diese Weise können wesentliche Bildeigenschaften in den Bilddaten selbst visualisiert werden, so dass letztlich ein visueller Beleg für das durch die Veränderungsinformation beschriebene Ergebnis an einen Benutzer ausgegeben wird. Insbesondere ist es denkbar, dass der Benutzer selbst gedanklich das Ergebnis des Auswertungsalgorithmus nochmals überprüfen kann oder zumindest die Auswertungsdarstellung in seine letztendliche Beurteilung des Eingriffserfolgs einbezieht.

In einer besonders bevorzugten Ausgestaltung kann vorgesehen sein, dass dann, wenn zu einem Patienten, für den eine Veränderungsinformation bestimmt wurde, eine Grundwahrheit vorliegt, dessen Bilddaten und die Grundwahrheit, sowie insbesondere eine Abweichungsinformation, als Trainingsdaten zum weiteren Trainieren des Algorithmus verwendet werden. Sobald mithin für einen Patienten bekannt ist, ob der Eingriff erfolgreich war oder nicht bzw. welche Veränderung an der Läsion aufgetreten ist, kann diese Grundwahrheit genutzt werden, um den Auswertungsalgorithmus weiter zu verbessern und somit ein ständig lernendes System zu realisieren, dessen Auswertungshilfen immer fundierter und verlässlicher werden.

Im Rahmen der Erfindung kann vorgesehen sein, dass das Trainieren des Auswertungsalgorithmus seitens einer zentralen, Trainingsdaten mehrerer medizinischer Infrastruktureinrichtungen nutzenden Backendeinrichtung erfolgt. Auf diese Weise können die Ergebnisse bzw. Grundwahrheiten und Bilddaten verschiedenster medizinischer Infrastruktureinrichtungen, insbesondere von Kliniken, zusammengeführt werden, um auf einer großen Datenbasis einen besonders hochqualitativen Auswertungsalgorithmus mit geeigneten Eingangsdaten zu schaffen. Dieser Auswertungsalgorithmus kann dann insbesondere den medizinischen Infrastruktureinrichtungen wieder bereitgestellt werden, die auch Trainingsdaten bereitgestellt haben. Eine zentrale Backendeinrichtung zum Trainieren hat ferner den Vorteil, dass dort eine große Rechenleistung dediziert für diesen Zweck herangezogen werden kann, die auch im Folgenden genutzt werden kann, um bei Vorliegen neuer Trainingsdaten, beispielsweise aufgrund von dem Auswertungsalgorithmus selbst beurteilten Fällen, Aktualisierungen vornehmen zu können und dergleichen. Selbstverständlich ist es grundsätzlich jedoch auch denkbar, eine Recheneinrichtung einer medizinischen Infrastruktureinrichtung einzusetzen, um dort einen Trainingsvorgang durchzuführen.

Es sei an dieser Stelle noch angemerkt, dass selbstverständlich in verschiedenen Koordinatensystemen vorliegende Bilddaten im Rahmen der vorliegenden Erfindung in ein gemeinsames Koordinatensystem überführt werden, was selbstverständlich auch für Bilddaten gilt, bei denen sich der Patient in unterschiedlichen Positionen befindet. Anders gesagt kann vorgesehen sein, dass in unterschiedlichen Koordinatensystemen und/oder Bewegungszuständen des Patienten aufgenommene Bilddaten miteinander registriert werden. Dies ermöglicht einen optimalen, implizit oder explizit durchzuführenden Vergleich von in Bildern sichtbaren Merkmalen und dergleichen. Insbesondere werden hierbei auch Bilddaten von unterschiedlichen Zeitpunkten in ein gemeinsames Koordinatensystem zusammengeführt. Die Registrierung kann dabei eine Intensitätsanpassung umfassen und/oder auf Landmarken basieren, beispielsweise übereinstimmenden anatomischen Strukturen und/oder Läsionen.

Neben dem Verfahren betrifft die Erfindung auch eine Auswertungseinrichtung mit wenigstens einem Rechenprozessor, ausgebildet zur Durchführung des erfindungsgemäßen Verfahrens. Sämtliche Ausführungen bezüglich des erfindungsgemäßen Verfahrens lassen sich analog auf die erfindungsgemäße Auswertungseinrichtung übertragen, so dass auch mit dieser die bereits genannten Vorteile erreicht werden können.

Die Auswertungseinrichtung kann hierzu insbesondere eine Trainingseinheit, eine Eigenschaftsdatenermittlungseinheit und eine Auswertungseinheit umfassen. Weitere Einheiten können eine Ausgabeeinheit, eine Registrierungseinheit, verschiedene Subeinheiten und dergleichen sein. Die Auswertungseinrichtung kann eine Kommunikationsschnittstelle zu einem Bildarchivierungssystem (PACS) und/oder zu einer Bildaufnahmeeinrichtung, insbesondere einer zur Bildüberwachung des Eingriffs genutzten Bildaufnahmeeinrichtung, und/oder einem Informationssystem, beispielsweise einem Krankenhausinformationssystem (HIS) und/oder einem Radiologieinformationssystem (RIS) aufweisen, um Bilddaten und/oder zur Ermittlung von Eigenschaftsdaten bzw. als weitere Eingangsdaten zu verwendende Informationen wenigstens teilweise abzurufen.

Ein erfindungsgemäßes Computerprogramm ist direkt in einen Speicher einer Auswertungseinrichtung ladbar und weist Programmmittel auf, um die Schritte eines hierin beschriebenen Verfahrens auszuführen, wenn das Computerprogramm in der Auswertungseinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, die zumindest ein genanntes Computerprogramm umfassen und derart ausgestaltet sind, dass sie bei Verwendung des Datenträgers in einer Auswertungseinrichtung ein hierin beschriebenes Verfahren durchführen. Bei dem Datenträger kann es sich um einen nichttransienten Datenträger, beispielsweise eine CD-ROM, handeln
Druckschrift US 2015/0087957 A1 betrifft ein Verfahren zur Beurteilung, ob eine Therapie bei einem Patienten, der beispielsweise an einem Tumor leidet, erfolgreich anschlägt respektive angeschlagen ist. Hierbei ist vorgesehen, dass zu Beginn der Therapie und im Rahmen weiterer Therapiesitzungen jeweils mittels einer medizinischen Bildgebungseinrichtung ein Bild des Tumors respektive der betroffenen Körperstelle erfasst wird. Die hierbei erzeugten Bilder werden sodann mittels einer künstlichen Intelligenz hinsichtlich ihrer Textureigenschaften derart ausgewertet, so dass eine Wahrscheinlichkeit, dass die Therapie erfolgreich wirkt, bestimmt wird.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: ein Zusammenhangsdiagramm zu einem Ausführungsbeispiel des erfindungsgemäßen Verfahrens, und
- Fig. 2: eine Prinzipskizze einer erfindungsgemäßen Auswertungseinrichtung.

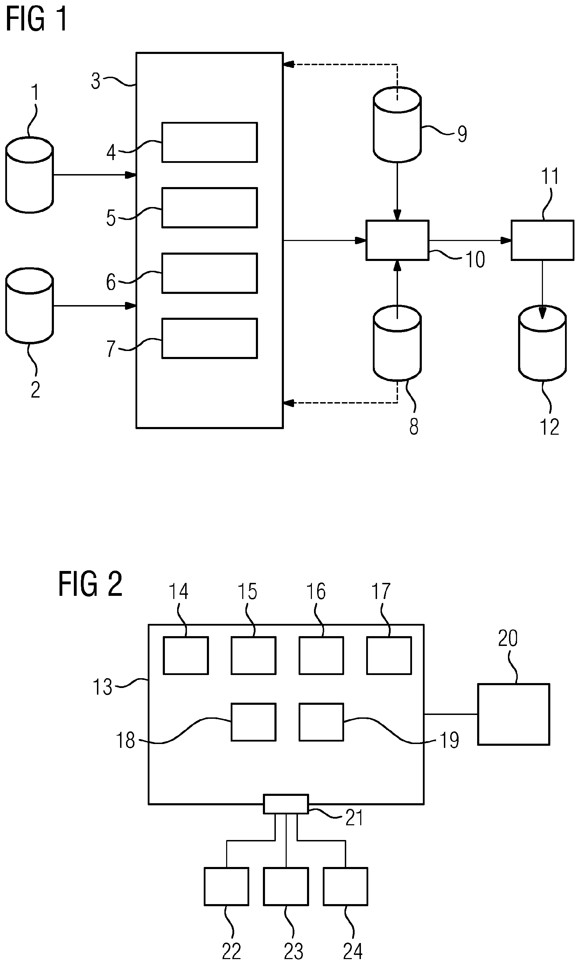

Fig. 1 zeigt eine Prinzipskizze zur Erläuterung eines Ausführungsbeispiels des erfindungsgemäßen Verfahrens, das vorliegend im Rahmen der Beurteilung des Erfolgs einer Tumorablation eingesetzt werden soll. Dabei wird als vorbereitende Bildaufnahmemodalität sowie als Modalität zur Bildüberwachung des minimalinvasiven Eingriffs, welcher hier mit einer Nadel als Instrument durchgeführt wird, Röntgen verwendet. Beispielsweise kann in einer Eingriffsräumlichkeit als Bildaufnahmeeinrichtung eine Röntgeneinrichtung mit einem C-Bogen verwendet werden, die auch CT-fähig ausgebildet sein kann. Vorbereitend und in Fig. 1 noch nicht näher abgebildet, findet jedoch zunächst ein Trainingsvorgang statt, in dem Trainingsdaten verwendet werden, um Eingangsdaten für einen Auswertungsalgorithmus und den Auswertungsalgorithmus selbst, der ein Algorithmus der künstlichen Intelligenz, hier ein Random-Forest-Algorithmus ist, zu bestimmen. Dabei wird ein Deep-Learning-Algorithmus eingesetzt, der unmittelbar auf in den Trainingsdaten enthaltenden Bilddaten ansetzt. Ziel ist ein Auswertungsalgorithmus, der eine Veränderungsinformation über die behandelte Läsion, hier einen Tumor, enthält, welche

wiederum benutzt werden kann, um den Erfolg des Eingriffs zu beurteilen. Beispielsweise kann ein Grad der Reduzierung der Läsion als Veränderungsinformation angestrebt werden und/oder eine Eigenschaftsveränderung oder dergleichen. In den Trainingsdaten ist den jeweiligen Bilddaten, die vorliegend einen präinterventionellen Bilddatensatz und einen intra- und/oder postinterventionellen Bilddatensatz enthalten, als Grundwahrheit insbesondere jeweils eine derartige Veränderungsinformation zugeordnet, wie sie der Auswertungsalgorithmus am Ende bestimmen soll. Der Deep-Learning-Trainingsalgorithmus erkennt die Zusammenhänge und wählt aus vorgegebenen, Eigenschaften der Bilddaten beschreibenden Eigenschaftsdaten geeignete Eigenschaftsdaten als Eingangsdaten des Auswertungsalgorithmus aus und/oder definiert selbst Eigenschaften, die über entsprechende Eigenschaftsdaten als Eingangsdaten in den Auswertungsalgorithmus eingehen können. Ferner liegen vorliegend als Trainingsdaten auch Eingriffsinformationen und Patienteninformationen vor, die ebenso als Eingangsdaten für den Auswertungsalgorithmus herangezogen werden können. Der Trainingsalgorithmus findet durch maschinelles Lernen die Zusammenhänge bzw. Korrelationen in den Bilddaten bzw. sonstigen potentiellen Eingangsdaten zu den Veränderungsinformationen heraus, parametriert den Auswertungsalgorithmus und definiert die Eingangsdaten, die dieser benötigt.

Dabei gilt selbstverständlich, dass auch jederzeit eine Aktualisierung stattfinden kann, mithin ein weiteres Training, wozu insbesondere tatsächlich beurteilte Eingriffe herangezogen werden können, zu denen zu einem späteren Zeitpunkt eine gesicherte Grundwahrheit vorliegt. Das Training des Auswertungsalgorithmus kann auf einer Backendeinrichtung, die Trainingsdaten verschiedener medizinischer Infrastruktureinrichtungen, beispielsweise Kliniken, nutzen kann, erfolgen.

Fig. 1 zeigt nun den konkreten Einsatz eines so bestimmten Auswertungsalgorithmus bei einem konkreten Eingriff an einem Patienten, so dass baldmöglichst während bzw. nach dem Eingriff der Erfolg des Eingriffs anhand der Veränderungsinformation beurteilt werden kann.

Um die Eingangsdaten für den Auswertungsalgorithmus zu bestimmen, wird auch im konkreten Fall von zwei Bilddatensätzen 1, 2 ausgegangen, nämlich einem präinterventionellen Bilddatensatz 1, hier einem CT-Datensatz, der zur Planung und für Überlagerungsdarstellungen während der Bildunterstützung des Eingriffs verwendet wurde, und einem intra- und/oder postinterventionellen Bilddatensatz 2, der vorliegend während und unmittelbar nach dem Eingriff aufgenommene Röntgenbilder bzw. hieraus rekonstruierte dreidimensionale Röntgenbilder als Bilddaten enthält. Eine das Verfahren durchführende Auswertungseinrichtung kann die Bilddatensätze 1, 2 dabei beispielsweise von einem über eine Kommunikationsschnittstelle angebundenen Bildarchivierungssystem (PACS), einer Bildaufnahmeeinrichtung direkt und/oder aus einem Informationssystem (HIS/RIS) erhalten.

Den jeweiligen Bilddaten der Bilddatensätze 1, 2 sind dabei auch Aufnahmeparameter zugeordnet, beispielsweise Aufnahmezeiten, Kontrastmitteleigenschaften, Röntgendosen, Röntgenenergiespektren und dergleichen, welche für den Auswertungsalgorithmus und/oder die vorangehende, nun zu beschreibende Ermittlung der Eigenschaftsdaten relevant sein können.

In einem Gesamtschritt 3, welcher, wie dargestellt, Unterschritte enthalten kann, werden die Bilddatensätze 1, 2 ausgewertet, um die angesprochenen Eigenschaftsdaten, die als Eingangsdaten für den Auswertungsalgorithmus verwendet werden sollen, zu ermitteln. In dem Gesamtschritt 3 kann auch ein Registrierungsschritt 4 enthalten sein, der in unterschiedlichen Koordinatensystemen und/oder unterschiedlichen Bewegungszuständen des Patienten aufgenommene Bilddaten in ein gemeinsames Koordinatensystem überführt.

Weitere Unterschritte befassen sich mit verschiedenen Arten, Eigenschaftsdaten zu ermitteln, wobei selbstverständlich die entsprechenden Unterschritte nur dann benötigt werden, wenn auch entsprechende Eigenschaftsdaten im Auswertungsalgorithmus tatsächlich eingesetzt werden sollen.

Ein Unterschritt 5 befasst sich mit der anatomischen Bildauswertung. Dabei werden Identifikations- und Positionsinformationen zu der Läsion und/oder die Läsion umgebenden anatomischen Strukturen mit im Stand der Technik grundsätzlich bekannten Algorithmen automatisch bestimmt. Hierbei können Segmentierungsalgorithmen, anatomische Atlanten und dergleichen beispielhaft eingesetzt werden. Vorliegend werden insbesondere auch potentielle Wärmesenken identifiziert, die Behandlungswärme abführen könnten, beispielsweise Blutgefäße und/oder Luftwege in der Lunge. Optional können biophysikalische Gewebeeigenschaften beschreibende Parameter ermittelt werden. Zweckmäßig ist es in jedem Fall, am Ende annotierte Regionen vorliegen zu haben.

Der Unterschritt 6 befasst sich mit der Bildauswertung der Bilddaten, in denen das verwendete Instrument, hier wie erwähnt eine Nadel, enthalten ist, um entsprechende Identifikations- und Positionsinformationen bezüglich dieses Instruments zu ermitteln, wobei wiederum grundsätzlich bekannte Algorithmen, beispielsweise Segmentierungsalgorithmen, eingesetzt werden können. In Eingriffsinformationen 9, auf die im Weiteren noch genauer eingegangen wird, können zudem auch bereits Geometrieinformationen zu dem medizinischen Instrument enthalten sein, die hier eingesetzt werden können, vgl. den entsprechenden gestrichelten Pfeil. In diesem Kontext sei auch angemerkt, dass auch später noch diskutierende Patientendaten 8 teilweise in Unterschritten 5, 6 berücksichtigt werden können.

Der Unterschritt 7 schließlich befasst sich mit der Ermittlung von abstraktere Eigenschaften betreffenden Eigenschaftsdaten, Form- und Textureigenschaften von Bilddaten. Diese beziehen sich auf verschiedene Bildmerkmale, beispielsweise Eigenschaften von Histogrammen, Umgebungsinformationen zu bestimmten Bildelementen, Fraktaleigenschaften und dergleichen. Auch durch den Trainingsalgorithmus selbst als nützlich definierte Eigenschaften betreffende Eigenschaftsdaten werden in diesem Unterschritt 7 ermittelt.

Es sei an dieser Stelle noch darauf hingewiesen, dass in diesem Ausführungsbeispiel auch der besonders vorteilhafte Effekt der Tatsache, dass die Bilddatensätze 1 und/oder 2 Spektral-CT-Daten, also Mehrenergie-Röntgendaten enthalten, genutzt wird. Aus solchen Multienergie-Röntgendaten lassen sich noch deutlich mehr nützliche Bildeigenschaften definieren und/oder ableiten, beispielsweise Kontrastmittel herausrechnen und dergleichen. Die Eigenschaftsdaten enthalten dabei jeweils, soweit sinnvoll, auch eine zeitliche Zuordnung (Aufnahmezeitpunkt der Bilddaten aus denen sie abgeleitet wurden) sowie eine räumliche Zuordnung als Einordnungsinformationen, so dass mithin innerhalb des Gesamtschritts 3 auch eine Art zeitliches und/oder räumliches Mapping der Eigenschaftsdaten erfolgen kann.

In einem Schritt 10 werden die Eingangsdaten für den in einem Schritt 1 zu nutzenden Auswertungsalgorithmus zusammengestellt. Die Eingangsdaten umfassen die im Gesamtschritt 3 ermittelten Eigenschaftsdaten und können zudem noch zumindest Anteile der Eingriffsdaten 9 und der Patientendaten 8 enthalten. Die Eingriffsdaten 9 beschreiben den minimalinvasiven Eingriff, können mithin Informationen wie Eingriffszeiten, eingesetzte Energie, gemessene Temperaturen, Instrumentengeometrie und dergleichen enthalten. Patientendaten 8 können beispielsweise Blutgerinnungseigenschaften, verabreichte Medikamente und dergleichen umfassen.

Nachdem alle Eingangsdaten für den Auswertungsalgorithmus der künstlichen Intelligenz zusammengestellt sind, wird dieser in einem Schritt 11 mit diesen Eingangsdaten ausgeführt, so dass die entsprechende wenigstens eine Veränderungsinformation 12 erhalten wird.

Die Veränderungsinformation 12 kann beispielsweise eine Klassifizierung des Eingriffsergebnisses in unterschiedliche Klassen (Residualtumor vorhanden, Tumor völlig entfernt und dergleichen) darstellen bzw. umfassen, so dass der Auswertungsalgorithmus in dieser Hinsicht auch als ein Klassifikator verstanden werden kann. Selbstverständlich können auch Erfolgswahrscheinlichkeiten, Veränderungsgrade und dergleichen als Veränderungsinformation bestimmt werden, die einem Benutzer helfen, die abschließende Beurteilung des Eingriffserfolgs unmittelbar nach dem Eingriff vorzunehmen.

Neben einer Darstellung der Veränderungsinformation 12 selber ist auch vorgesehen, eine Auswertungsdarstellung zu erzeugen, in welcher wichtige Eigenschaftsdaten Bilddaten überlagert dargestellt werden, so dass ein visueller Beleg für die Veränderungsinformation für den Benutzer erzeugt werden kann.

Liegt zu dem hier beispielhaft begleitenden minimalinvasiven Eingriff zu einem späteren Zeitpunkt, beispielsweise aus einem später aufgenommenen Bilddatensatz oder dergleichen, eine Grundwahrheit über den Erfolg des minimalinvasiven Eingriffs vor, können die entsprechenden Bilddaten gemeinsam mit weiteren Eingangsdaten des Auswertungsalgorithmus und der Grundwahrheit sowie gegebenenfalls eine Abweichungsinformation, die die Abweichung der Grundwahrheit von der Veränderungsinformation, die bestimmt wurde, betrifft, als weitere Trainingsdaten verwendet werden, um den Auswertungsalgorithmus zu aktualisieren.

Fig. 2 zeigt eine stark abstrahierte Prinzipskizze einer erfindungsgemäßen Auswertungseinrichtung 13, mit der das erfindungsgemäße Verfahren ausgeführt werden kann. Diese weist vorliegend eine Trainingseinheit 14 zum Trainieren des Auswertungsalgorithmus, eine Registrierungseinheit 15 zur Registrierung von Bilddaten, eine Eigenschaftsdatenermittlungseinheit 16 zur Durchführung des Gesamtschrittes 3, eine Aggregationseinheit 17 zur Zusammenstellung der Eingangsdaten (Schritt 10), eine Auswertungseinheit 18 zur Realisierung des Auswertungsalgorithmus und eine Ausgabeeinheit 19 zur Ausgabe der Veränderungsinformation 12, daraus abgeleiteter Informationen und/oder der Auswertungsdarstellung, wofür beispielsweise ein entsprechendes Ausgabemittel 20 angesteuert werden kann, auf.

Über eine Kommunikationsschnittstelle 21, die unter anderem die Aggregationseinheit 17 nutzen kann, besteht Zugriff auf verschiedene externe Datenquellen, beispielsweise ein Bildarchivierungssystem 22, ein Informationssystem 23 und den Speicher einer Bildaufnahmeeinrichtung 24.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Auswertung von Bilddaten eines Patienten nach einem minimalinvasiven Eingriff an einer Läsion, insbesondere einer Tumorablation an einem Tumor, zur Entscheidung über minimalinvasive Korrekturmaßnahmen wie ablative Maßnahmen, wobei ein präinterventioneller, die Läsion zeigender Bilddatensatz (1) und ein intra- und/oder postinterventioneller zweiter, das Eingriffsgebiet zeigender Bilddatensatz (2) ausgewertet werden, wobei aus den Bilddatensätzen (1, 2) wenigstens teilweise automatisch Eigenschaften der Bilddaten beschreibende Eigenschaftsdaten abgeleitet werden, welche einem mit Trainingsdaten, denen eine Grundwahrheit zugeordnet ist, anderer Patienten trainierten Auswertungsalgorithmus der künstlichen Intelligenz als Eingangsdaten bereitgestellt werden, welcher aus den Eigenschaftsdaten eine die Veränderung der Läsion und/oder im Eingriffsgebiet durch den Eingriff beschreibende Veränderungsinformation (12) ermittelt, wobei aus der Veränderungsinformation eine wenigstens einem Teil der Bilddaten überlagert darzustellende Auswertungsdarstellung erzeugt und ausgegeben wird.

2. Verfahren nach Anspruch 1, **dadurch gekennzeichnet, dass** als Auswertungsalgorithmus ein neuronales Netz und/oder ein Random-Forest-Algorithmus verwendet werden.

3. Verfahren nach Anspruch 1 oder 2, **dadurch gekennzeichnet, dass** wenigstens ein Teil der als Eingangsdaten zu verwendenden Eigenschaftsdaten und/oder Eigenschaften des Auswertungsalgorithmus durch einen den Auswertungsalgorithmus trainierenden Trainingsalgorithmus, insbesondere einen Deep-Learning-Algorithmus, definiert werden.

4. Verfahren nach einem der vorangehenden Ansprüche, **dadurch gekennzeichnet, dass** wenigstens ein Teil der als Eingangsdaten zu verwendenden Eigenschaftsdaten aus einer vorgegebenen Menge an Kandidateneigenschaften beschreibenden Kandidatendaten ausgewählt werden.

5. Verfahren nach einem der vorangehenden Ansprüche, **dadurch gekennzeichnet, dass** als Eingangsdaten des Auswertungsalgorithmus und/oder zur Ermittlung der Eigenschaftsdaten zusätzlich den Bilddatensätzen (1, 2) zugeordnete, zur Aufnahme von Bilddaten der Bilddatensätze (1, 2) verwendete Aufnahmeparameter verwendet werden.

6. Verfahren nach Anspruch 5, **dadurch gekennzeichnet, dass** wenigstens ein Teil der Bilddaten Multienergie-Röntgenbilddaten mit zugeordneten Energiespektreninformationen umfasst.

7. Verfahren nach einem der vorangehenden Ansprüche, **dadurch gekennzeichnet, dass** als Eigenschaftsdaten Identifikations- und Positionsinformationen zu der Läsion und/oder die Läsion umgebenden anatomischen Strukturen, insbesondere als annotierte Regionen, und/oder ein verwendetes, in den Bilddaten sichtbares Instrument betreffende Instrumentinformationen ermittelt werden.

8. Verfahren nach einem der vorangehenden Ansprüche, **dadurch gekennzeichnet, dass** als Eigenschaftsdaten Form- und Textureigenschaften der Bilddaten beschreibende Eigenschaftsdaten ermittelt werden.

9. Verfahren nach einem der vorangehenden Ansprüche, **dadurch gekennzeichnet, dass** als Eingangsdaten des Auswertungsalgorithmus und/oder zur Ermittlung der Eigenschaftsdaten zusätzlich den Patienten betreffende Patienteninformationen (8) und/oder Diagnoseinformationen und/oder den Eingriff beschreibende Eingriffsinformationen (9) verwendet werden.

10. Verfahren nach einem der vorangehenden Ansprüche, **dadurch gekennzeichnet, dass** den Eigenschaftsdaten und/oder weiteren Eingangsdaten jeweils eine zeitliche und/oder räumliche Einordnung erlaubende Einordnungsinformationen zugeordnet werden.

11. Verfahren nach einem der vorangehenden Ansprüche, **dadurch gekennzeichnet, dass** aus wenigstens einem Teil der Eigenschaftsdaten eine wenigstens einem Teil der Bilddaten überlagert darzustellende Auswertungsdarstellung erzeugt und ausgegeben wird.

12. Verfahren nach einem der vorangehenden Ansprüche, **dadurch gekennzeichnet, dass** dann, wenn zu einem Patienten, für den eine Veränderungsinformation bestimmt wurde, eine Grundwahrheit vorliegt, dessen Bilddatensätze (1, 2) und die Grundwahrheit, sowie insbesondere eine Abweichungsinformation, als Trainingsdaten zum weiteren Trainieren des Algorithmus verwendet werden.

13. Auswertungseinrichtung (13) mit wenigstens einem Rechenprozessor und einem Ausgabemittel (20), wobei der Rechenprozessor zur Durchführung eines Verfahrens nach einem der vorangehenden Ansprüche ausgebildet ist und wobei das Ausgabemittel (20) zur Ausgabe von einer durch das Verfahren erzeugten Auswertungsdarstellung angepasst ist.

14. Computerprogramm, welches die Schritte eines Verfahrens nach einem der Ansprüche 1 bis 12 durchführt, wenn es auf einer Recheneinrichtung ausgeführt wird.

15. Elektronisch lesbarer Datenträger, auf dem ein Computerprogramm nach Anspruch 14 gespeichert ist.

## Claims

1. Method for analyzing image data from a patient after a minimally invasive intervention on a lesion, in particular after a tumor ablation on a tumor, for deciding on minimally invasive correction measures such as ablative measures, wherein a pre-intervention image dataset (1) showing the lesion and an intra- and/or post-intervention, second image dataset (2) showing the intervention region are analyzed, wherein attribute data describing attributes of the image data is derived from the image datasets (1, 2) at least in part automatically, which attribute data is provided as input data to an artificial intelligence analysis algorithm that is trained using training data from other patients, which training data is associated with a ground truth, and that determines from the attribute data, change information (12) that describes the change in the lesion and/or in the intervention region as a result of the intervention, wherein an analysis representation to be presented as an overlay on at least some of the image data is generated from the change information and is output.

2. Method according to claim 1, **characterised in that** a neural network and/or a random forest algorithm is used as the analysis algorithm.

3. Method according to claim 1 or claim 2, **characterised in that** at least some of the attribute data and/or attributes of the analysis algorithm, which are to be used as input data, are defined by a training algorithm, in particular a deep-learning algorithm, training the analysis algorithm.

4. Method according to one of the preceding claims, **characterised in that** at least some of the attribute data to be used as input data is selected from a predetermined set of candidate data describing candidate attributes.

5. Method according to one of the preceding claims, **characterised in that** acquisition parameters associated with the image datasets (1, 2) and used for acquiring image data of the image datasets (1, 2) are used in addition as input data of the analysis algorithm and/or for determining the attribute data.

6. Method according to claim 5, **characterised in that** at least some of the image data comprises multi-energy radiographic data containing associated energy-spectrum information.

7. Method according to one of the preceding claims, **characterised in that** identification and position information relating to the lesion and/or to the anatomical structures surrounding the lesion, in particular as annotated regions, and/or instrument information relating to an instrument that is used and is visible in the image data, is determined as the attribute data.

8. Method according to one of the preceding claims, **characterised in that** attribute data describing shape and texture attributes of the image data is determined as the attribute data.

9. Method according to one of the preceding claims, **characterised in that** patient information (8) and/or diagnostic information relating to the patient and/or intervention information (9) describing the intervention is used in addition as input data of the analysis algorithm and/or for determining the attribute data.

10. Method according to one of the preceding claims, **characterised in that** classification information allowing a temporal and/or spatial classification is assigned to each item of attribute data and/or additional input data.

11. Method according to one of the preceding claims, **characterised in that** an analysis representation to be presented as an overlay on at least some of the image data is generated from at least some of the attribute data, and is output.

12. Method according to one of the preceding claims, **characterised in that** when a ground truth exists for a patient for whom change information has been determined, image datasets (1, 2) from said patient and the ground truth, and in particular divergence information, is used as training data for further training of the algorithm.

13. Analysis apparatus (13) comprising at least one processor and an output means (20), wherein the processor is designed for performing a method according to one of the previous claims and wherein the output means (20) is adjusted to output from an analysis representation generated by the method.

14. Computer program, which performs the steps of a method according to one of claims 1 to 12 when it is executed in a processing device.

15. Electronically readable data storage medium, on which is stored a computer program according to claim 14.

## Revendications

1. Procédé d'exploitation de données d'image d'un patient après une intervention invasive au minimum sur une lésion, notamment une ablation d'une tumeur, pour décider de mesures de correction invasives au minimum, comme des mesures d'ablation, dans lequel on exploite un ensemble (1) de données d'image avant l'intervention montrant la lésion et un deuxième ensemble (2) de données d'image intra interventionnel et/ou postinterventionnel montrant la région de l'intervention, dans lequel on déduit des ensembles (1, 2) de données d'image des données de propriété, qui décrivent automatiquement, au moins en partie, des propriétés des données d'image et qui sont procurées, comme données d'entrée, à un algorithme d'exploitation de l'intelligence artificielle ayant subi un apprentissage par des données d'apprentissage, auxquelles est associée une grande véracité, d'autres patients, qui détermine à partir des données de propriété une information (12) de modification décrivant la modification de la lésion et/ou dans la région d'intervention par l'intervention, dans lequel on produit à partir de l'information de modification une représentation d'exploitation, représentant en superposition au moins une partie des données d'image et on l'émet.

2. Procédé suivant la revendication 1, **caractérisé en ce que** l'on utilise comme algorithme d'exploitation un réseau neuronal et/ou un algorithme random-forest.

3. Procédé suivant la revendication 1 ou 2, **caractérisé en ce que** l'on définit au moins une partie des données de propriété à utiliser comme données d'entrée et/ou des données de propriété de l'algorithme d'exploitation par un algorithme d'apprentissage faisant subir un apprentissage à l'algorithme d'exploitation, notamment par un algorithme deep-learning.

4. Procédé suivant l'une des revendications précédentes, **caractérisé en ce que** l'on choisit au moins une partie des données de propriété à utiliser comme données d'entrée dans un ensemble de données candidates décrivant des propriétés candidates.

5. Procédé suivant l'une des revendications précédentes, **caractérisé en ce que** l'on utilise, comme données d'entrée de l'algorithme d'exploitation et/ou pour la détermination des données de propriété, des paramètres d'enregistrement associés supplémentairement aux ensembles (1, 2) de données d'image et utilisés pour l'enregistrement de données d'image des ensembles (1, 2) de données d'image.

6. Procédé suivant la revendication 5, **caractérisé en ce qu'**on au moins une partie des données d'image comprend des données d'image aux rayons X à énergies multiples, ayant des informations associées de spectre d'énergie.

7. Procédé suivant l'une des revendications précédentes, **caractérisé en ce que** l'on détermine, comme données de propriété, des informations d'identification et de position de la lésion et/ou entourant la lésion, notamment sous la forme de régions annotées et/ou des informations d'instrument concernant un instrument utilisé, visible dans les données d'image.

8. Procédé suivant l'une des revendications précédentes, **caractérisé en ce que** l'on détermine, comme données de propriété, des données de propriété décrivant des propriétés de forme et de texture des données d'image.

9. Procédé suivant l'une des revendications précédentes, **caractérisé en ce que** l'on utilise, comme données d'entrée de l'algorithme d'exploitation et/ou pour la détermination des données de propriété, supplémentairement des informations (8) de patient concernant le patient et/ou des informations de diagnostic et/ou des informations (9) d'intervention décrivant l'intervention.

10. Procédé suivant l'une des revendications précédentes, **caractérisé en ce que** l'on associe aux données de propriété et/ou à d'autres données d'entrée, respectivement des informations de classement, permettant un classement temporel et/ou spatial.

11. Procédé suivant l'une des revendications précédentes, **caractérisé en ce que** l'on produit, à partir d'au moins une partie des données de propriété, une représentation d'exploitation représentant en superposition une partie des données d'image et on l'émet.

12. Procédé suivant l'une des revendications précédentes, **caractérisé en ce que**, lorsqu'on a, pour un patient pour lequel une information de modification a été déterminée, une grande véracité, on utilise ses ensembles (1, 2) de données d'image et la grande véracité, ainsi que notamment une information d'écart, comme données d'apprentissage pour continuer à faire subir un apprentissage à l'algorithme.

13. Dispositif (13) d'exploitation, comprenant au moins un processeur informatique et un moyen (20) de sortie, le processeur informatique étant constitué pour effectuer un procédé suivant l'une des revendications précédentes et le moyen (20) de sortie étant propre à sortir une représentation d'exploitation produite par le procédé.

14. Programme d'ordinateur qui effectue les stades d'un procédé suivant l'une des revendications 1 à 12, lorsqu'il est réalisé sur un dispositif informatique.

15. Support de données déchiffrables électroniquement, sur lequel est mis en mémoire un programme d'ordinateur suivant la revendication 14.
